(19) Europäisches Patentamt
European Patent Office
Office européen des brevets

(11) Veröffentlichungsnummer : **0 402 755 B1**

# (12) EUROPÄISCHE PATENTSCHRIFT

(45) Veröffentlichungstag der Patentschrift :
**25.08.93 Patentblatt 93/34**

(51) Int. Cl.$^5$ : **C07C 229/76,** C07F 3/02, A61K 31/195, A61K 31/28

(21) Anmeldenummer : **90110725.0**

(22) Anmeldetag : **06.06.90**

## (54) L-Carnitin-Magnesium-Zitrat.

(30) Priorität : **14.06.89 CH 2221/89**

(43) Veröffentlichungstag der Anmeldung :
**19.12.90 Patentblatt 90/51**

(45) Bekanntmachung des Hinweises auf die Patenterteilung :
**25.08.93 Patentblatt 93/34**

(84) Benannte Vertragsstaaten :
**AT BE CH DE DK ES FR GB IT LI LU NL SE**

(56) Entgegenhaltungen :
**EP-A- 0 150 688**
**FR-M- 4 608**
**GB-A- 1 153 640**

(73) Patentinhaber : **LONZA AG**
**Gampel/Wallis Geschäftsleitung Basel**
**CH-4002 Basel (CH)**

(72) Erfinder : **Scholl, Thomas, Dr,. Chemiker**
**Terbinerstrasse 40**
**VISP (Kanton Wallis) (CH)**
Erfinder : **Kohl, Willibald, Dr., Dipl.-Ing.**
**Gurtenweg 29A**
**Muri b.Bern (Kanton Bern) (CH)**

(74) Vertreter : **Weinhold, Peter, Dr. et al**
**Patentanwälte Dipl.-Ing. G. Dannenberg Dr. P. Weinhold Dr. D. Gudel Dipl.-Ing. S. Schubert Dr. P. Barz Siegfriedstrasse 8**
**D-80803 München (DE)**

EP 0 402 755 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft L-Carnitin-Magnesium-Zitrat, welches als echtes Komplexsalz vorliegt, ein Verfahren zur Herstellung dieser Verbindung und die Verwendung als Kombinationspräparat von Carnitin und Magnesium in der Sporternährung oder als pharmakologischer Wirkstoff. Diese Verbindung hat die folgende Formel

$$(CH_3)_3\overset{\oplus}{N}-CH_2-\underset{\displaystyle OH}{\underset{|}{CH}}-CH_2-COOH \quad \cdot \quad \begin{matrix} H_2C-COO^{\ominus} \\ | \\ HO-C-COO^{\ominus} \\ | \\ H_2C-COO^{\ominus} \end{matrix} \quad \cdot \quad Mg^{2+}$$

Sowohl Magnesium als auch L-Carnitin werden vermehrt beim körperlichen Training über Schweiss bzw. Urin ausgeschieden. Diese Verluste bewirken:

- Magnesium- bzw. L-Carnitin-Mangelerscheinungen
- Muskelkrämpfe
- Verminderung der Leistungsfähigkeit
- Herzrhythmusstörungen.

Bekannt ist, dass L-Carnitin sowie die Magnesiumsalze, Magnesium-aspartat und Magnesium-orotat eine hohe Hygroskopizität aufweisen.

Die Aufgabe der vorliegenden Erfindung bestand darin, ein neues Derivat des L-Carnitins herzustellen, welches eine geringe Hygroskopizität und eine gute Hitzestabilität aufweist. Erfindungsgemäss wird dies durch die Patentansprüche 1 bis 10 erreicht.

Das Verfahren zur Herstellung von L-Carnitin-Magnesium-Zitrat kann erfindungsgemäss aus stöchiometrischen Anteilen einer Magnesiumverbindung, Zitronensäure und L-Carnitin, in einem geeigneten Lösungsmittel, wie beispielsweise in Wasser, Methanol und Ethanol, durchgeführt werden. Vorzugsweise wird die Reaktion in einem wässrigen Medium durchgeführt. Die Reaktionstemperatur liegt zweckmässig bei 20 bis 100°C, vorzugsweise bei 50 bis 70°C.

Als Magnesiumverbindungen können Magnesiumhydroxid, Magnesiumoxid und Magnesiumchlorid angewendet werden, vorzugsweise wird Magnesiumhydroxid angewendet.

Nach einem weiteren erfindungsgemässen Verfahren kann L-Carnitin-Magnesium-Zitrat aus Magnesium-Zitrat und L-Carnitin gewonnen werden.

Das Salz wird erhalten, wenn die Lösung nach einer gewissen Reaktionszeit entweder sprühgetrocknet, vakuumgetrocknet, gefriergetrocknet oder am Rotationsverdampfer eingeengt wird, vorzugsweise wird die Lösung durch Sprühtrocknen eingeengt.

Durch Sprühtrocknen wird das gewünschte Produkt in der gewünschten Korngrösse erhalten. Anstelle von Sprühtrocknen kann die Lösung am Rotationsverdampfer eingeengt werden und der anfallende Rückstand durch Reinigungsbehandlung in einem geeigneten Lösungsmittel weiterbehandelt werden. Zweckmässig wird der Rückstand in einer Mischung aus einem niedrig siedenden Alkohol mit einem aliphatischen Keton aufgenommen. Als Alkohol können Methanol, Ethanol, Propanol und Isopropanol angewendet werden, vorzugsweise wird Methanol angewendet. Als Keton können Aceton und Methyl-ethyl-Keton verwendet werden, vorzugsweise, wird Aceton verwendet.

Die Verbindung der vorliegenden Erfindung stellt ein ideales Verhältnis von L-Carnitin und Magnesium dar. Ein gesunder Erwachsener enthält im Muskelgewebe sowohl 20 g Magnesium als auch 20 g L-Carnitin und kann zur optimalen Energieversorgung 2 g L-Carnitin und um 300 mg Magnesium aufnehmen. Eine tägliche Dosis von 2 bis 5 g L-carnitin-Magnesium-Zitrat versorgt den Körper mit 780 bis 1950 mg L-Carnitin und mit 126 bis 315 mg Magnesium. Durch den synergistischen Effekt von Magnesium und L-Carnitin weist die Verbindung der vorliegenden Erfindung ausserordentliche, nützliche Eigenschaften auf:

- deutlich höhere Leistungen und verbesserte Ausdauer bei Sportlern sowie kürzere Erholungszeiten
- Ausgleich des erhöhten Magnesium- und L-Carnitin-Bedarfs beim Leistungssport
- verzögerte Ermüdung
- Stärkung der Herzleistung und Verhinderung von Herzrhythmusstörungen
- erhöhte Stresstoleranz
- geringere Neigung zu Muskel- und Gefässkrämpfe
- Verbesserung der Muskeltätigkeit
- Erhöhung der Aktivität von Enzymreaktionen im Energiestoffwechsel

Beispiel

L-Carnitin-Magnesium-Zitrat

Ein Gemisch von Zitronensäure (19,3 g, 0,1 mol), Magnesiumhydroxid (6,1 g, 0,1 mol) und L-Carnitin (16,1 g, 0,1 mol) wurden in Wasser (50 ml) gelöst und 1 Stunde bei 60°C gerührt. Durch Sprühtrocknen wurden aus der klaren Lösung 36,0 g Produkt als feines, leichtes Pulver erhalten, entsprechend einer Ausbeute von 95% (bezogen auf eingesetztes L-Carnitin).

Wurde die Lösung am Rotationsverdampfer eingeengt und anschliessend der Rückstand in einer Mischung aus Aceton (100 ml) und Methanol (100 ml) aufgenommen, dann filtriert und getrocknet (12 Stunden bei 60°C und 40 mbar), erhielt man ein gröberes Pulver, entsprechend einer Ausbeute von 95% (bezogen auf eingesetztes L-Carnitin).

Schmelzpunkt: über 250°C
Spez. Drehung: $[\alpha]_D^{25}$ [c = 1% in $H_2O$] - 12° (± 1°)
Löslichkeit: über 50 g/100 ml Wasser
Die Struktur wurde durch IR-, NMR- und Röntgen-Spektroskopie bestätigt.

Hitzestabilität:

Nach 24 Stunden an der Luft bei 100°C äusserlich unverändert, Gewichtsabnahme 5%

Hygroskopizität:

| | | | | | | |
|---|---|---|---|---|---|---|
| Luftfeuchtigkeit % | 32 | 44 | 56 | 66 | 73 | 80 |
| Wasseraufnahme % nach 1 Woche | 8 | 15 | 21 | 29 | 35 | 46 |

Kein Zerfliessen

**Patentansprüche**

1. L-Carnitin-Magnesium-Zitrat.

2. Verfahren zur Herstellung von L-Carnitin-Magnesium-Zitrat der Formel

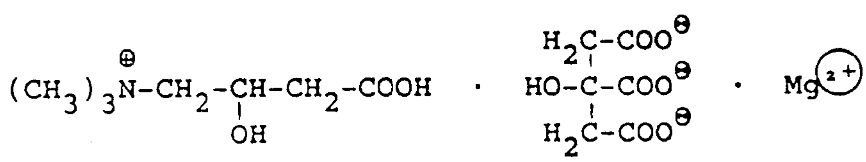

nach Patentanspruch 1, dadurch gekennzeichnet, dass man eine Magnesiumverbindung, Zitronensäure und L-Carnitin in stöchiometrischen Anteilen, in einem geeigneten Lösungsmittel, zu dem entsprechenden L-Carnitin-Magnesium-Zitrat umsetzt und anschliessend aus der Lösung das L-Carnitin-Magnesium-Zitrat gewinnt.

3. Verfahren zur Herstellung von L-Carnitin-Magnesium-Zitrat nach Patentanspruch 1, dadurch gekennzeichnet, dass man Magnesium-Zitrat und L-Carnitin in stöchiometrischen Anteilen, in einem geeigneten Lösungsmittel, zu dem entsprechenden L-Carnitin-Magnesium-Zitrat umsetzt und anschliessend aus der Lösung das L-Carnitin-Magnesium-Zitrat gewinnt.

4. Verfahren nach mindestens einem der Patentansprüche 2 bis 3, dadurch gekennzeichnet, dass man als Lösungsmittel Wasser verwendet.

5. Verfahren nach mindestens einem der Patentansprüche 2 und 4, dadurch gekennzeichnet, dass man als Magnesiumverbindung Magnesiumhydroxid, Magnesiumoxid oder Magnesiumchlorid verwendet.

6. Verfahren nach mindestens einem der Patentansprüche 2, 4 und 5, dadurch gekennzeichnet, dass man als Magnesiumverbindung Magnesiumhydroxid verwendet.

7. Verfahren nach mindestens einem der Patentansprüche 2 bis 6, dadurch gekennzeichnet, dass man das L-Carnitin-Magnesium-Zitrat aus der Lösung durch Einengen bis zur Trockene gewinnt.

8. Verfahren nach Patentanspruch 7, dadurch gekennzeichnet, dass man die Lösung durch Sprühtrocknen einengt.

9. Verfahren nach Patentanspruch 7, dadurch gekennzeichnet, dass man die Lösung am Rotationsverdampfer einengt.

10. Verfahren nach mindestens einem der Patentansprüche 7 und 9, dadurch gekennzeichnet, dass man, nach Einengen am Rotationsverdampfer L-Carnitin-Magnesium-Zitrat, in einem Gemisch von einem niedrig siedenden Alkohol mit einem aliphatischen Keton umkristallisiert.

11. Verwendung von L-Carnitin-Magnesium-Zitrat als Kombinationspräparat von Carnitin und Magnesium in der Sporternährung.

12. L-Carnitin-Magnesium-Zitrat zur Anwendung als pharmakologischer Wirkstoff.

## Claims

1. L-carnitine magnesium citrate.

2. A process for the preparation of L-carnitine magnesium citrate of the formula

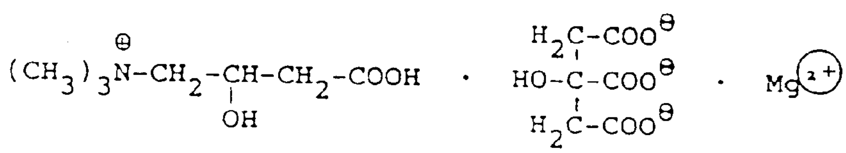

according to claim 1, characterized in that a magnesium compound, citric acid and L-carnithine in stoichiometric amounts are reacted in a suitable solvent to the corresponding L-carnitine magnesium citrate and that then the L-carnitine magnesium citrate is obtained from the solution.

3. A process for the preparation of L-carnitine magnesium citrate accordin to claim 1, characterized in that a magnesium citrate and L-carnitine in stoichiometric amounts are reacted in a suitable solvent to the corresponding L-carnitine magnesium citrate and that then the L-carnitine magnesium citrate is obtained from the solution.

4. A process according to at least one of the claims 2 to 3, characterized in that water is used as solvent.

5. A process according to at least one of the claims 2 and 4, characterized in that magnesium hydroxide, magnesium oxide or magnesium chloride is used as magnesium compound.

6. A process according to at least one of the claims 2, 4 and 5, characterized in that magnesium hydroxide is used as magnesium compound.

7. A process according to at least one of the claims 2 to 6, characterised in that the L-carnitine magnesium citrate is obtained from the solution by concentrating it to dryness.

8. A process according to claim 7, characterized in that the solution is concentrated by spray drying.

9. A process according to claim 7, characterized in that the solution is concentrated at the rotatory evaporator.

10. A process according to at least one of the claims 7 and 9, characterized in that the L-carnitine magnesium citrate, after concentrating it on the rotatory evaporator, is recrystallized in a mixture of a low-boiling alcohol and an aliphatic ketone.

11. The use of L-carnitine magnesium citrate as combination preparation of carnitine and magnesium in sport nutrition.

12. L-carnitine magnesium citrate to be used as pharmacological active substance.

## Revendications

1. Composé de L-carnitine magnésium citrate.

2. Procédé pour la préparation de composé de L-carnitine magnésium citrate selon la formule

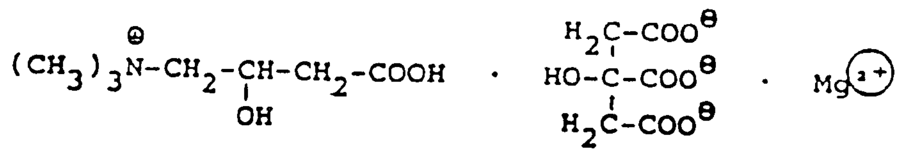

selon la revendication 1, caractérisé en ce que l'on transforme un composé de magnésium d'acide citrique et de L-carnitine en proportions stoechiométriques, dans un solvant approprié, en le composé correspondant de L-carnitine magnésium citrate, puis à partir de la solution on obtient le composé de L-carnitine magnésium citrate.

3. Procédé pour la préparation de L-carnitine magnésium citrate selon la revendication 1, caractérisé en ce que l'on transforme le magnésium citrate et la L-carnitine dans des proportions stoechiométriques, dans un solvant approprié, en le composé correspondant de L-carnitine magnésium citrate et on obtient ensuite à partir de la solution le composé L-carnitine magnésium citrate.

4. Procédé selon au moins l'une des revendications 2 à 3, caractérisé en ce que l'on utilise de l'eau en tant que solvant.

5. Procédé selon au moins l'une des revendications 2 et 4, caractérisé en ce que l'on utilise comme composé de magnésium l'hydroxyde de magnésium, l'oxyde de magnésium ou le chlorure de magnésium.

6. Procédé selon au moins l'une des revendications 2, 4 et 5, caractérisé en ce qu'en tant que composé de magnésium, on utilisé l'hydroxyde de magnésium.

7. Procédé selon au moins l'une des revendications 2 à 6, caractérisé en ce que l'on obtient le composé L-carnitine magnésium citrate à partir de la solution par concentration jusqu'à siccité.

8. Procédé selon la revendication 7, caractérisé en ce que l'on concentre la solution par séchage par pulvérisation.

9. Procédé selon la revendication 7, caractérisé en ce que l'on concentre la solution dans un évaporateur rotatif.

10. Procédé selon au moins l'une des revendications 7 et 9, caractérisé en ce qu'après concentration dans un évaporateur rotatif, on recristallise le composé L-carnitine magnésium citrate, dans un mélange d'un alcool à faible point d'ébullition avec une cétone aliphatique.

11. Utilisation de L-carnitine magnésium citrate en tant que préparation combinée de carnitine et de magnésium dans l'alimentation sportive.

12. L-carnitine magnésium citrate pour l'application en tant que substance pharmacologique active.